# EUROPÄISCHE PATENTSCHRIFT

(11) **EP 0 447 844 B1**
(45) Veröffentlichungstag und Bekanntmachung des Hinweises auf die Patenterteilung: **23.11.1994**
(21) Anmeldenummer: 91102883.5
(22) Anmeldetag: 27.02.1991
(51) Int. Cl.: A61M 16/00, A61M 16/06

(54) **Vorrichtung zur Oxygenierung eines Patienten**
Oxygenation device for a patient
Dispositif pour oxygéner un malade

(30) Priorität: 21.03.1990 DE 4009008
(43) Veröffentlichungstag der Anmeldung: 25.09.1991
(73) Patentinhaber: Zander, Rolf, Prof.Dr., D-55124 Mainz (DE); Mertzlufft, Friedrich, Dr., D-55126 Mainz (DE)
(72) Erfinder: Zander, Rolf, Prof.Dr., D-55124 Mainz (DE); Mertzlufft, Friedrich, Dr., D-55126 Mainz (DE)
(74) Vertreter: Weber, Dieter, Dr.

(56) Entgegenhaltungen:
- DE-A- 3 719 009
- DE-C- 356 630
- US-A- 3 037 501

## Beschreibung

Warmblüterzellen sind auf kontinuierliche und ausreichende, bedarfsorientierte Zufuhr von Sauerstoff angewiesen, so daß bereits kurze Unterbrechungen der Sauerstoffzufuhr irreparable Schäden in und an den Zellen, insbesondere des Zentralnervensystems und des Herz-Kreislaus-Systems, hervorrufen können.

Sauerstoffmangel, Hypoxie, kann prinzipiell durch mangelhafte Zufuhr, Störungen der Lungenfunktion, Störungen des Transportes im Blut oder Mangel an Hämoglobin sowie durch Störungen des Herz-Kreislauf-Systems hervorgerufen werden. In der Notfallmedizin wie auch bei der innerklinischen Versorgung ist es daher Standard, einem Patienten Sauerstoff zuzuführen. Im Rahmen der sogenannten Präoxygenierung, vor allem vor jeder Intubation, ist es zwingend erforderlich, den Sauerstoffbedarf des Patienten in der gefährlichen Zeitspanne bis zur Beendigung der Intubation oder für den Fall von Intubationskomplikationen zu decken.

In solchen und anderen Fällen ist es unerläßlich, den Speicherraum der Lunge mit reinem Sauerstoff anzureichern und gleichzeitig auch sicherzustellen, daß andere sich im Körper oder in der Einatemluft befindliche Gase, vor allem Stickstoff, möglichst vollständig entfernt werden. Dies gelingt nach üblichen Präoxygenierungsmethoden meistens gar nicht oder nur sehr unzureichend. Bekannte Vorrichtungen zur Oxygenierung sind Masken, Rachenröhren und Nasen-Rachen-Röhren, sogenannte Tuben.

Eine Beatmungsmaske ist in der DE-A-3 719 009 beschrieben.

Die Nachteile dieser Vorrichtungen, in der Literatur beschrieben (z. B. R. G. Sandersen [ed.]: The Cardiac Patient, Philadelphia, W. B. Saunders, 1972, Seite 310), sind vor allem
- mangelnde Anfeuchtung und Erwärmung des Gases,
- Sauerstoffkonzentration niemals 100 %,
- Rückatmung von Ausatemluft (CO₂- und N₂-Anreicherung),
- Undichtigkeit des Systems (Aufnahme von N₂) und
- sehr großer Sauerstoff-Flow.

Die der Erfindung zugrundeliegende Aufgabe bestand somit darin, eine Vorrichtung zur Versorgung eines Patienten mit Sauerstoff (Oxygenierung) zu bekommen, mit Hilfe derer gleichzeitig mit der Sauerstoffanreicherung unerwünschte andere Gase möglichst vollständig aus dem Lungenraum entfernt werden.

Zusätzlich sollten die oben genannten Nachteile bekannter Vorrichtungen eliminiert werden.

Erfindungsgemäß ist diese Vorrichtung zur Oxygenierung eines Patienten mit einem Sauerstoffapplikator für die Zufuhr von Sauerstoff über die Nase dadurch gekennzeichnet, daß der Sauerstoffapplikator so ausgebildet ist, daß er reinen Sauerstoff in gerichtetem Fluß (nasoral) ausschließlich über die Nase (nasal) zuführt und daß die Vorrichtung zusätzlich ein in den Mund (oral) des Patienten wenigstens im wesentlichen dichtend einsetzbares Einwegventil aufweist, das nur bei Gasausstrom öffnet und ansonsten schließt und überschüssigen Sauerstoff und Ausatemluft nur über den Mund entweichen läßt.

Prinzipiell kann mit dieser Vorrichtung erreicht werden, daß einerseits
- ein gerichteter Fluß, nämlich Trennung von Einatmung (100 % O₂ über die Nase) und Ausatmung (überschüssiger O₂, N₂ und CO₂ über das Mundventil, d. h. "nas-oral", erzwungen wird,
andererseits
- ein physiologischer Fluß, nämlich Anfeuchtung und Erwärmung über die Nase, ohne Vergrößerung des Totraumes und Vermeidung einr Rückatmung, eingehalten wird.

Mit dieser Vorrichtung wird sichergestellt, daß bei bestehender oder sistierender Atmung (Apnoe) des Patienten nur reiner Sauerstoff (erwärmt und angefeuchtet) in die Atemwege und Lungen gelangen kann und alle zu eliminierenden Gase (Stickstoff, Lachgas, Kohlendioxid) vollständig (N₂, N₂O) oder physiologisch (CO₂) über das Mundventil abgegeben werden.

Der Patient kann mit dieser Vorrichtung selbst bei fehlender Eigenatmung (sogenannte apnoische Oxygenierung) nur noch reinen Sauerstoff "inhalieren", da das Einwegventil das unkontrollierte und unerwünschte Eindringen von Außenluft und damit großer Mengen an Stickstoff verhindert. Dies funktioniert zeitlich unbegrenzt. Sobald ein Atemstillstand eingetreten ist, erwünscht (zur Intubation) oder unerwünscht (Notfall), kann das Mundventil entfernt werden, wenn sichergestellt ist, daß überschüssiger Sauerstoff über den Mund entweicht. In diesem Zustand können nun therapeutische Maßnahmen über den Mund vorgenommen werden (z. B. Absaugen, Intubieren, Blutstillung), aber auch diagnostische Maßnahmen sind möglich (z. B. Bronchoskopie, Laryngoskopie). Arzt, Schwester oder Sanitäter haben zusätzlich beide Hände frei für eventuell erforderliche Routine- oder Akutmaßnahmen, wie z. B. im Notfall das Herrichten der Instrumente zur künstlichen Beatmung und der entsprechenden Medikamente oder auch das Legen eines periphervenösen Zugangs zur Verabreichung von lebensrettenden oder auch anästhesiologisch zwingend erforderlichen Medikamenten oder Blut bzw. Blutersatzstoffen. Auch haben Arzt, Laienhelfer oder Sanitäter beide Hände frei für Maßnahmen der Wiederbelebung, wie z. B. extrathorakale Herzmassage, Defibrillation, Injektionen oder Infusionen. Ein weiterer Vorteil der Vorrichtung besteht darin, daß sie es selbst dem Laien gestattet, den vorhandenen, schon unter Normalbedingungen minimalen Sauerstoffvorrat signifikant und risikolos zu steigern, was im gesamten Bereich der außerklinischen und innerklinischen Patientenversorgung für die tägliche Routineanwendung von großem Nutzen ist. Eine Mithilfe des Patienten ist nicht erforderlich. Die Vorrichtung kann so mit Vorteil bei Hypoxie unterschiedlicher Ursache oder zur Präoxygenierung vor Intubationen eingesetzt werden. Bei diesen vermindert die erfindungsgemäße Vorrichtung a priori das Gesamtrisiko, das insbesondere durch mögliche Intubationshindernisse besteht.

Die gleiche Vorrichtung ist universell einsetzbar, nämlich sowohl in der Notfallmedizin (wacher oder bewußtloser Patient, vorhandene oder sistierende Atmung) als auch in der Anästhesiologie (präoperativ: Präoxygenierung, postoperativ: Aufwachraum), in der Intensivmedizin (Respirator-Entwöhnung), in der Hals-Nasen-Ohren-Heilkunde (Laryngoskopie), in der Pulmologie (Bronchoskopie) und in der Zahn-, Mund- und Kiefer-Heilkunde (Oral-Chirurgie).

Wesentlich an der erfindungsgemäßen Vorrichtung ist, daß die Einführung des Sauerstoffes ausschließlich über die Nase erfolgt. Der Sauerstoffüberlauf und die Ausatmung gehen durch den Mund, der durch das Einwegventil an der Einatmung gehindert ist.

Der Sauerstoffapplikator kann unterschiedlich ausgebildet sein und in seinem zuführenden Teil beispielsweise in Form eines Nasenkatheters vorliegen. Zweckmäßig verwendet man aber einen Sauerstoffapplikator in Form einer Maske, die nur die Nase bedeckt. Vorzugsweise ist diese Maske so ausgebildet, daß sie im wesentlichen dichtend an die Nase anlegbar ist.

Der Sauerstoffapplikator kann in an sich bekannter Weise mit einem Sauerstoffbeutel verbunden sein, der zwischen die Sauerstoffquelle und den Sauerstoffapplikator zwischengeschaltet ist und optisch den Einatmungsvorgang beobachten läßt. Zusätzlich oder anstelle des Sauerstoffbeutels kann an dem Sauerstoffapplikator und/oder an dem Einwegventil ein akustisches oder optisches Signal angebracht werden, das ausgelöst wird, wenn die erwünschte Einatmung oder Ausatmung erfolgt bzw. bei Gaseinstrom oder Gasausstrom.

Zur einfachen und universellen (innerklinisch wie außerklnisch) Handhabung des Sauerstoffbeutels empfiehlt sich folgende Anordnung: Der Sauerstoffbeutel am Sauerstoffschlauch 2 des Sauerstoffapplikators 1 weist einen Konnektor vom Durchmesser 22 mm (DIN-ISO 5356 Teil I) auf, welcher sowohl mit dem Sauerstoffapplikator 1 als auch mit einem Intubationstubus direkt verbunden werden kann, zusätzlich über einen Konnektor vom Durchmesser 15 mm (DIN-ISO 7228) sowohl mit dem an die zentrale Sauerstoffversorgung fest angeschlossenen Ventilations-System als auch über den in diese Verbindung integrierten Konnektor nach ISO 594/1 mit einer Sauerstoff-Flasche.

Diese Anordnung eröffnet die Möglichkeit, nach endotrachealer Intubation (Einwegventil 3 entfernt) die Oxygenierung bzw. Denitrogenisierung des Patienten aufrechtzuerhalten (Transport vom Narkose-Einleitungsraum zum OP bzw. vom OP zum Aufwachraum oder zur Intensivstation, Lagerungs- bzw. Umlagerungs-Maßnahmen), nachdem der Sauerstoffbeutel mit einer Klemme verschlossen und vom Sauerstoffschlauch 2 dekonnektiert wurde (mobile Oxygenierung).

Wie oben ausgeführt, ist das Einwegentil so ausgebildet, daß es im wesentlichen dichtend in den Mund des Patienten einsetzbar ist. Hierzu ist das Einwegventil zweckmäßig mit einer elastischen Dichtungsplatte versehen, die dem Patienten vor dem Ober- und Unterkiefer zwischen diesen und den Lippen eingefügt wird. Besonders zweckmäßig schließt sich an diese Dichtungsplatte ein Rohrstutzen an, der in den Rachenraum ragt und auf den der Patient zweckmäßig beißt, um die Dichtungsplatte in dichtender Lage zu halten.

Die Dichtung des Einwegventils kann aber selbstverständlich auch auf andere Weise erfolgen, wie beispielsweise durch einen gegebenenfalls aufblasbaren Gummiballon, der sich beim Aufblasen dichtend in die Mundöffnung legt.

Die beiden zur erfindungsgemäßen Vorrichtung gehörenden Einrichtungen, Sauerstoffapplikator und Einwegventil, können getrennt voneinander als Vorrichtungssatz vorliegen. Zweckmäßig aber sind sie miteinander verbunden, so daß der Anwender, der Arzt, die Schwester oder der Sanitäter, beide Einrichtungen stets griffbereit zusammen vorliegen hat.

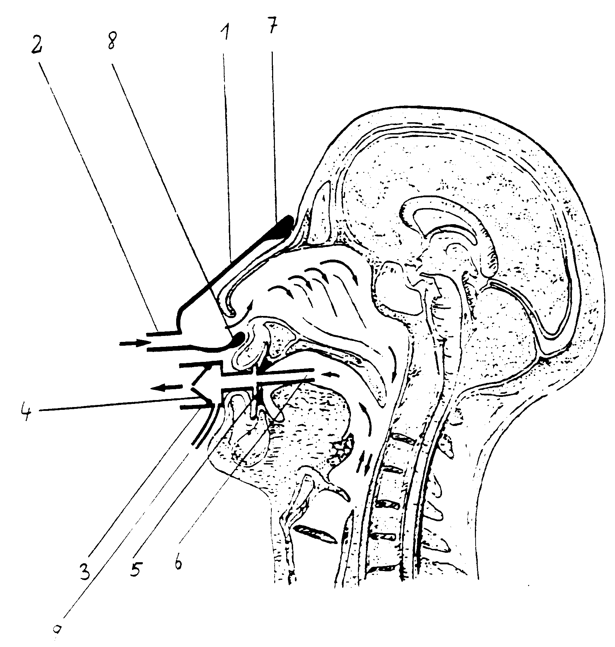

In der Zeichnung ist eine Ausführungsform der Vorrichtung nach der Erfindung schematisch senkrecht im Schnitt dargestellt.

Die erfindungsgemäße Vorrichtung besteht aus dem Sauerstoffapplikator 1 und dem Einwegventil 3. Die Sauerstoffapplikator-Einrichtung 1 besitzt die Form einer Maske mit zwei Dichtlippen 7 und 8, die sich oberhalb und unterhalb der Nase anlegen und eine Sauerstoffzuführung nur über die Nase gestatten. Der Sauerstoffapplikator 1 ist mit einem Sauerstoffschlauch 2 verbunden, der, gegebenenfalls über einen Sauerstoffbeutel, mit einer Sauerstoff-Flasche in Verbindung steht.

Das Einwegventil 3 besitzt eine elastische Dichtungsplatte 5, die zwischen den Lippen und Kiefern (Zähnen) des Patienten im wesentlichen dichtend einfügbar ist. An diese Dichtungsplatte 5 schließt sich ein Rohrstutzen 6 an, auf den der Patient beißt. In dem erweiterten vorderen Teil des Einwegventils, das außerhalb des Mundes liegt, finden sich die Klappen 4, die so ausgebildet sind, daß sie sich beim Ausatmen bzw. beim Gasausstrom auseinanderspreizen, während sie ansonsten (z. B. beim Einatmungsvorgang) dichtend aneinandergepreßt werden und das Eindringen von Außenluft verhindern.

Unter klinischen Bedingungen kann sowohl die Denitrogenisierung als auch die Ventilation eines Patienten über endexspiratorische Gasproben kontrolliert werden, und zwar mittels Massenspektroskopie (O₂, CO₂, N₂) oder Kapnometrie (O₂, CO₂): Zu diesem Zwecke findet sich am Einwegventil 3 ein spezieller, verschließbarer Gasauslaßstutzen 9 zum Anschluß entsprechender Meßsysteme (ISO 594/1) und zur kontinuierlichen Gasprobennahme (Nebenstrom-Prinzip).

## Patentansprüche

1. Vorrichtung zur Oxygenierung eines Patienten mit einem Sauerstoffapplikator für die Zufuhr von Sauerstoff über die Nase und mit einem Ventil, **dadurch gekennzeichnet**, daß der Sauerstoffapplikator (1) so ausgebildet ist, daß er reinen Sauerstoff in gerichtetem Fluß ausschließlich über die Nase zuführen kann und daß das Ventil der Vorrichtung ein in den Mund des Patienten wenigstens im wesentlichen dichtend einsetzbares Einwegventil (3) ist, das nur bei Gasausstrom öffnet und ansonsten schließt und überschüssigen Sauerstoff und Ausatemluft nur über den Mund entweichen läßt.

2. Vorrichtung nach Anspruch 1, **dadurch gekennzeichnet**, daß der Sauerstoffapplikator (1) als nur mit der Nase verbundene Maske ausgebildet ist.

3. Vorrichtung nach Anspruch 1 oder 2, **dadurch gekennzeichnet**, daß das Einwegventil (3) eine zwischen den Lippen und den Kiefern bzw. Zähnen des Patienten einfügbare elastische Dichtungsplatte (5) aufweist.

4. Vorrichtung nach Anspruch 3, **dadurch gekennzeichnet**, daß das Einwegventil (3) einen sich an die Dichtungsplatte (5) anschließenden Rohrstutzen (6) aufweist.

5. Vorrichtung nach einem der Ansprüche 1 bis 4, **dadurch gekennzeichnet**, daß das Einwegventil (3) elastische Plättchen aufweist, die nur bei Gasausstrom auseinandergespreizt und ansonsten (z. B. beim Einatmen) im wesentlichen dichtend gegeneinander gepreßt werden.

6. Vorrichtung nach einem der Ansprüche 1 bis 5, **dadurch** **gekennzeichnet**, daß der Sauerstoffapplikator (1) und das Einwegventil (3) miteinander verbunden sind.

7. Vorrichtung nach einem der Ansprüche 1 bis 6, **dadurch gekennzeichnet**, daß das Einwegventil (3) für die Dauer des Öffnens ein optisches oder akustisches Signal erzeugt.

8. Vorrichtung nach einem der Ansprüche 1 bis 6, **dadurch gekennzeichnet**, daß das Einwegventil (3) jeweils beim Öffnen und Schließen ein optisches oder akustisches Signal erzeugt.

9. Vorrichtung nach einem der Ansprüche 1 bis 6, **dadurch gekennzeichnet**, daß das Einwegventil (3) einen verschließbaren Gasauslaßstutzen (9) enthält (6 mm Durchmesser Konnektionsverbindung gemäß ISO 594/1) zur kontinuierlichen, endexspiratorischen Gasanalyse.

10. Vorrichtung nach einem der Ansprüche 1 bis 2, **dadurch gekennzeichnet**, daß der Sauerstoffschlauch (2) des Sauerstoffapplikators (1) eine Konnektionsverbindung mit einem Durchmesser von 22 mm aufweist (DIN-ISO 5356 Teil I).

11. Vorrichtung nach einem der Ansprüche 1 bis 2, **dadurch gekennzeichnet**, daß der Sauerstoffschlauch (2) des Sauerstoffapplikators (1) einen Konnektor gemäß ISO 594/1 aufweist, der in eine Konnektionsverbindung gemäß DIN-ISO 7228 integriert ist.

## Claims

1. Apparatus for oxygenating a patient having an oxygen administration means for the supply of oxygen by way of the nose and having a valve characterised in that the oxygen administration means (1) is so designed that it can supply pure oxygen in a directed flow exclusively by way of the nose and that the valve of the apparatus is a one-way valve (3) which can be at least substantially sealingly fitted into the mouth of the patient and which opens only with a gas discharge flow and otherwise closes and allows excess oxygen and exhalation air to escape only by way of the mouth.

2. Apparatus according to claim 1 characterised in that the oxygen administration means (1) is in the form of a mask which is connected only to the nose.

3. Apparatus according to claim 1 or claim 2 characterised in that the one-way valve (3) has an elastic sealing plate (5) which can be inserted between the lips and the jawbones or teeth of the patient.

4. Apparatus according to claim 3 characterised in that the one-way valve (3) has a tubular connecting portion (6) adjoining the sealing plate (5).

5. Apparatus according to one of claims 1 to 4 characterised in that the one-way valve (3) has elastic plate portions which are spread apart only in the event of a gas discharge flow and otherwise (for example when the patient breathes in) are pressed substantially sealingly against each other.

6. Apparatus according to one of claims 1 to 5 characterised in that the oxygen administration means (1) and the one-way valve (3) are connected together.

7. Apparatus according to one of claims 1 to 6 characterised in that the one-way valve (3) produces an optical or acoustic signal for the duration of its opening.

8. Apparatus according to one of claims 1 to 6 characterised in that the one-way valve (3) produces an optical or acoustic signal whenever it opens and closes.

9. Apparatus according to one of claims 1 to 6 characterised in that the one-way valve (3) has a closable gas outlet connection (9) (6 mm diameter communication connection in accordance with ISO 594/1) for continuous endexpiratory gas analysis.

10. Apparatus according to one of claims 1 and 2 characterised in that the oxygen hose (2) of the oxygen administration means (1) has a communication connection of a diameter of 22 mm (DIN-ISO 5356 Part I).

11. Apparatus according to one of claims 1 and 2 characterised in that the oxygen hose (2) of the oxygen administration means (1) has a connector in accordance with ISO 594/1 which is integrated into a communication connection in accordance with DIN-ISO 7228.

## Revendications

1. Dispositif destiné à oxygéner un patient, comportant un applicateur d'oxygène pour l'amenée d'oxygène par l'intermédiaire du nez, ainsi qu'une soupape, caractérisé en ce que l'applicateur d'oxygène (1) est réalisé d'une manière telle qu'il puisse amener de l'oxygène pur, selon un flux orienté, exclusivement par l'intermédiaire du nez, et en ce que la soupape du dispositif est une soupape à une voie (3) susceptible d'être insérée au moins sensiblement étanche dans la bouche du patient, la soupape n'ouvrant que lors d'une sortie de gaz, fermant par ailleurs, et ne laissant s'échapper de l'oxygène excédentaire et de l'air d'expiration, que par la bouche.

2. Dispositif selon la revendication 1, caractérisé en ce que l'applicateur d'oxygène (1) est réalisé sous la forme d'un masque uniquement relié au nez.

3. Dispositif selon la revendication 1 ou 2, caractérisé en ce que la soupape à une voie (3) comporte une plaque d'étanchéité (5) élastique, susceptible d'être insérée entre les lèvres et les mâchoires ou les dents du patient.

4. Dispositif selon la revendication 3, caractérisé en ce que la soupape à une voie (3) comporte une tubulure (6) se raccordant à la plaque d'étanchéité (5).

5. Dispositif selon l'une des revendications 1 à 4, caractérisé en ce que la soupape à une voie (3) comprend des plaquettes élastiques, qui s'écartent l'une de l'autre uniquement en cas d'échappement de gaz, et qui en-dehors de cela (par exemple lors de l'inspiration), sont pressées l'une contre l'autre, de manière sensiblement étanche.

6. Dispositif selon l'une des revendications 1 à 5, caractérisé en ce que l'applicateur d'oxygène (1) et la soupape à une voie (3) sont reliés l'un à l'autre.

7. Dispositif selon l'une des revendications 1 à 6, caractérisé en ce que la soupape à une voie (3) engendre un signal optique ou acoustique pendant la durée de l'ouverture.

8. Dispositif selon l'une des revendications 1 à 6, caractérisé en ce que la soupape à une voie (3) engendre un signal optique ou acoustique lors de chaque ouverture et fermeture.

9. Dispositif selon l'une des revendications 1 à 6, caractérisé en ce que la soupape à une voie (3) comprend une tubulure de gaz (9) (raccord de connexion de 6 mm de diamètre selon ISO 594/1), destiné à l'analyse continue des gaz de fin d'expiration.

10. Dispositif selon l'une des revendications 1 à 2, caractérisé en ce que le tuyau souple d'oxygène (2) de l'applicateur d'oxygène (1), comporte un raccord de connexion d'un diamètre de 22 mm (DIN-ISO 5356 partie I).

11. Dispositif selon l'une des revendications 1 à 2, caractérisé en ce que le tuyau souple d'oxygène (2) de l'applicateur d'oxygène (1), comporte un connecteur selon ISO 594/1, qui est intégré dans un raccord de connexion selon DIN-ISO 7228.
